# EUROPÄISCHE PATENTSCHRIFT

(11) **EP 1 339 382 B1**
(45) Veröffentlichungstag und Bekanntmachung des Hinweises auf die Patenterteilung: **25.02.2015**
(21) Anmeldenummer: 01994742.3
(22) Anmeldetag: 28.11.2001
(51) Int. Cl.: C08L 51/00, C08F 291/00, C08F 290/06, C08F 265/04, C08F 271/02, A61Q 1/10, A61K 8/81

(54) **DEKORATIVE KOSMETISCHE UND DERMATOLOGISCHE MITTEL**
DECORATIVE COSMETIC AND DERMATOLOGICAL PRODUCTS
AGENTS COSMETIQUES ET DERMATOLOGIQUES DECORATIFS

(30) Priorität: 01.12.2000 DE 10059818
(43) Veröffentlichungstag der Anmeldung: 03.09.2003
(73) Patentinhaber: Clariant Produkte (Deutschland) GmbH, 65929 Frankfurt am Main (DE)
(72) Erfinder: LÖFFLER, Matthias, 65527 Niedernhausen (DE); MORSCHHÄUSER, Roman, 55122 Mainz (DE)
(86) Internationale Anmeldenummer: PCT/EP2001/013866
(87) Internationale Veröffentlichungsnummer: WO 2002/043688

(56) Entgegenhaltungen:
- EP-A- 0 356 241
- EP-A- 0 815 828
- EP-A- 0 815 844
- EP-A- 0 815 845
- WO-A-00/12588
- FR-A- 2 791 558
- US-A- 3 931 089
- US-A- 4 521 578
- US-A- 5 368 850
- US-A- 6 054 138
- "Surfactant free-emulsion cosmetics-comprising water, oil component and emulsion and consisting of water-insoluble and water-absorbable polymer" WPI WORLD PATENT INFORMATION DERWENT, DERWENT, GB, Bd. 29, Nr. 83, XP002029282

## Beschreibung

Dekorative kosmetische Mittel stehen in verschiedenen Formen, beilspielsweise Pudern, Suspensionen, Dispersionen, Cremes und Gelen zur Verfügung. Bevorzugt sind Emulsionen, sowohl als O/W- als auch als W/O-Emulsionen. Emulsionen werden im allgemeinen mit anionischen, nicht-ionischen und/oder amphoteren Emulgatoren hergestellt. Derartige klassische emulgatorhaltige Emulsionen führen oftmals, insbesondere bei langandauernder und häufiger Anwendung, zu Irritationen der Haut. Bei O/W-Emulsionen werden als Gelierungsmittel für die wässrige Phase beispielsweise Polyquaterniumverbindungen eingesetzt. Unbefriedigend ist das Aufnahmevermögen der Pigmente in der Gelphase und die Feinteiligkeit bzw. Stabilität der Emulsionen. Vorteilhaft für kosmetische Emulsionen sind Wasser-in-Öl Emulsionen, die eine hautglättende und feuchtigkeitsspendende Wirkung besitzen und der Haut ein gutes Erscheinungsbild geben. In W/O-Emulsionen werden die eingesetzten Pigmente durch Coating oder andere Modifizierungen in die Ölphase überführt. Um ein ausreichende Haftung der kosmetischen Mittel auf der Haut zu erreichen werden hydrophobe Filmbildner, beispielsweise alkylierte Vinylpyrrolidonpolymere, zugesetzt. Die hydrophoben Filmbildner werden jedoch durch das sich bildende Hautfett gelöst, wodurch die Haftung der Mittel abnimmt-zudem können unerwünschte Farbverschiebungen der Pigmente auftreten.

Bei einer weiteren bekannten Herstellweise von dekorativen kosmetischen Mittel werden hydrophile Filmbildner der externen Wasserphase von Öl-in-Wasser Emulsionen zugesetzt. Nachteilig dabei ist, dass die Farben vor und nach dem Auftragen der Mittel sehr unterschiedlich sind.

DE 19 907 587 und WO 9 904 750 beschreiben die Verwendung in kosmetischen Polymermitteln von Zusammensetzungen auf Basis von Acryloyldimethyltauraten.

Überraschenderweise wurde nun gefunden, dass eine neue Klasse von Copolymeren auf Basis von Acryloyldimethyltaurinsäure - die sowohl als Verdicker, Konsistenzgeber, Emulgator, Filmbildner, Haftmittel, Gleitmittel, Dispergator und/oder Stabilisator dienen können sich hervorragend zur Formulierung einer Vielzahl von dekorativen kosmetischen Mitteln eignen.

Gegenstand der Erfindung ist die Verwendung von kosmetischen und dermatologischen Mitteln, dadurch gekennzeichnet, dass sie mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) einer oder mehreren mindestens monofunktionellen, zur radikalischen Polymerisation befähigten, siliziumhaltigen Komponente(n), wobei mindestens eine siliziumhaltige Komponente eine Verbindung ausgewählt aus den Formeln 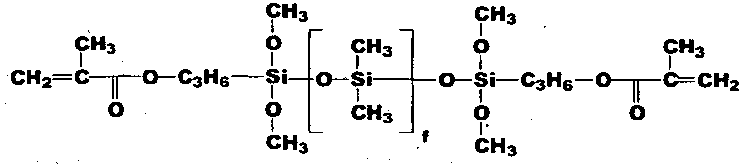 worin f = 2 bis 500 bedeutet, 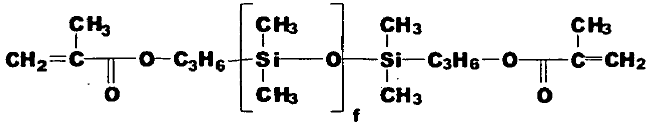 worin f = 2 bis 500 bedeutet, und 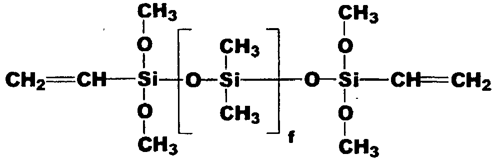 worin f = 2-500 bedeutet, ist,
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation in Gegenwart mindestens eines polymeren Additivs mit zahlenmittleren Molekulargewichten von 200 g/mol bis 10⁹ g/mol ausgewählt aus Polyvinylpyrrolidonen, Poly(N-Vinylformamiden), Poly(N-Vinylcaprolactemen) und Copolymeren aus N-Vinylpyrrolidon, N-Vinylformamid und/oder Acrylsäure, erfolgt,
enthalten, als dekorative Mittel.

Die erfindungsgemäßen Copolymere besitzen bevorzugt ein Molekulargewicht von bevorzugt 5*10⁴ bis 5*10⁶ g/mol.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituentewder Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80%.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.

Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.

Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.

Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁ - C₂₂)-Alkylreste oder 3 (C₂ - C₁₀)-Hydroxyalkylreste handeln kann. Zusätzlich können auch ein bis dreifach ethoxylierte

Ammoniumverbindungen mit unterschiedlichem Ethoxylierungsgrad Anwendung finden. Der Neutralisationsgrad der Carbonsäuren kann zwischen 0 und 100% betragen.

Als Comonomere B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropyl-methacrylamid und Bernsteinsäuremono-[2-(methacryloyloxy)-ethylester]; N,N-Dimethylamino-methacrylat; Diethylamino-methylmethacrylat; Acryl- und Methacrylamidoglykolsäure; 2- und 4-Vinylpyridin; Vinylacetat; Methacrylsäureglycidyläster; Styrol; Acrylnitril; Vinylchlorid; Stearylacrylat; Laurylmethacrylat; Vinylidenchlorid; und/oder Tetrafluorethylen.

Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen, zwitterionischen, oder amphoteren Struktur tragen.

Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphomeren Derivate überführt werden können.

Besonders bevorzugt als Comonomere C) sind
Diallyldimethylammoniumchlorid (DADMAC),
[2-((Methacryloyloxy)ethyl]trimethylammoniumchlorid (MAPTAC),
[2-((Acryloyloxy)ethyl]trimethylammoniumchlorid,
[2-Methacrylamidoethyl]trimethylammoniumchlorid,
[2-((Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain.

Der Gewichtsanteil der Comonomeren C) kann, bezogen auf die Gesamtmasse der Copolymere, 0,1 bis 99,8 Gew.-%, bevorzugt 0,5 bis 30 Gew.-%, insbesondere bevorzugt 1 bis 20 Gew.-%, betragen.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte silikonhaltige Komponenten sind solche gemäß Formel (I).

R¹ - Z- [(Si(R³R⁴)-O-)_{w}-(Si(R⁵R⁶)-O)ₓ-]- R² (I)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.

Zur Anbindung der silikonhaltigen Polymerkette an die reaktive Endgruppe R¹ ist eine geeignete chemische Brücke Z erforderlich. Bevorzugte Brücken Z sind -O-, -((C₁ - C₅₀)Alkylen)-, -((C₆ - C₃₀) Arylen)-, -((C₅ - C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückengruppierungen Z sind -((C₁ - C₁₀)Alkyl)-(Si(OCH₃)₂)- und -(Si(OCH₃)₂)-.

Der polymere Mittelteil wird durch silikonhaltige Wiederholungseinheiten repräsentiert.

Die Reste R³, R⁴, R⁵ und R⁶ bedeuten unabhängig voneinander -CH₃, -O-CH₃, -C₆H₅ oder -O-C₆H₅.

Die Indizes w und x repräsentieren stöchiometrische Koeffizienten, die unabhängig voneinander 0 bis 500, bevorzugt 10 bis 250, betragen.

Die Verteilung der Wiederholungseinheiten über die Kette hinweg kann nicht nur rein statistisch, sondern auch blockartig, alternierend oder gradientenartig sein kann.

R² kann einerseits einen aliphatischen, olefinischen, cycloaliphatischen, arylaliphatischen oder aromatischen (C₁-C₅₀)-Kohlenwasserstoffrest symbolisieren (linear oder verzweigt) oder -OH, -NH₂, -N(CH₃)₂, -R⁷ oder für die Struktureinheit [-Z-R¹] stehen. Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph).

Wenn R² ein Element der Gruppe [-Z-R¹] darstellt, handelt es sich um difunktionelle, Monomere, die zur Vernetzung der entstehenden Polymerstrukturen herangezogen werden können.

Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Als siliziumhaltige Komponenten (D) sind alle mindestens einfach definisch ungesättigten Verbindungen geeignet, wobei mindestens eine siliziumhaltige Komponente eine Verbindung ausgewählt aus den Formeln 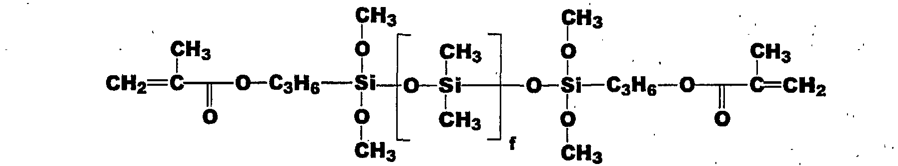 Methacryloxyproplydimethylsilyl endgeblockte Polydimethylsiloxane (f=2 bis 500) 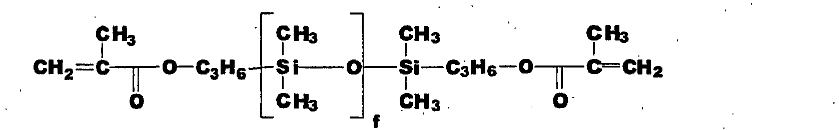 Methacryloxypropyl endgeblockte Polydimethylsiloxane (f= 2 bis 500 bis) 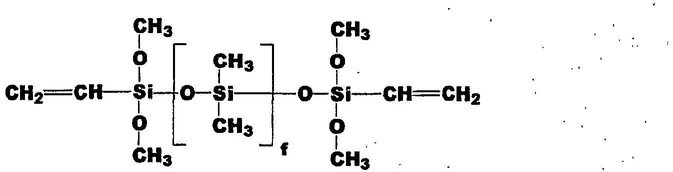 Vinyldimethoxysilyl endgeblockte Polydimethylsiloxane (f=2-500).

Bezogen auf die Gesamtmasse der Copolymere kann der Gehalt an siliziumhaltigen Komponenten bis 99,9 Gew.-%, bevorzugt 0,5 bis 30 Gew.-%, insbesondere bevorzugt, 1 bis 20 Gew.-%.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri-, oder polyfunktionelle Komponenten E) zu zumindest teilvernetzten Strukturen führen.

Bevorzugte fluorhaltige Komponenten E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH_{3]}-CO-), Crotonyl-, Senecionyl-, Itaconyl-, Maleinyl-, Fumaryl- oder Styrylrest, besonders bevorzugt einen Acryl- und Methacrylrest, dar.

Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.

Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99.9 Gew.-%, bevorzugt 0.5 bis 30 Gew.-%, insbesondere bevorzugt 1 bis 20 Gew.-%, betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen, die aus einer oder mehreren Wiederholungseinheit(en) aufgebaut sind und eine für Polymere charakteristische Molekulargewichtsverteilung aufweisen.

Bevorzugt als Makromonomere F) sind Verbindungen gemäß Formel (III).

R¹ - Y - [(A)ᵥ - (B)_{w} - (C)ₓ - (D)_{z}] - R² (III)

R¹ stellt eine polymerisationsfähige Funktion aus der Gruppe der vinylisch ungesättigten Verbindungen dar, die zum Aufbau polymerer Strukturen auf radikalischem Wege geeignet sind. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.

Zur Anbindung der Polymerkette an die reaktive Endgruppe ist eine geeignete verbrückende Gruppe Y erforderlich. Bevorzugte Brücken Y sind -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -O-.

Der polymere Mittelteil des Makromohomeren wird durch die diskreten Wiederholungseinheiten A, B, C und D repräsentiert. Bevorzugte Wiederholungseinheiten A,B,C und D leiten sich ab von Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.

Die Indizes v, w, x und z in Formel (III) repräsentieren die stöchiometrische Koeffizienten betreffend die Wiederholungseinheiten A, B, C und D. v, w, x und z betragen unabhängig voneinander 0 bis 500, bevorzugt 1 bis 30, wobei die Summe der vier Koeffizienten im Mittel ≥ 1 sein muss.

Die Verteilung der Wiederholungseinheiten über die Makromonomerkette kann statistisch, blockartig, alternierend oder gradientenartig sein.

R² bedeutet einen linearen oder verzweigten aliphatischen, olefinischen, cycloaliphatischen, arylaliphatischen oder aromatischen (C₁-C₅₀)-Kohlenwasserstoffrest, OH, -NH₂, -N(CH₃)₂ oder ist gleich der Struktureinheit [-Y-R¹].

Im Falle von R² gleich [-Y-R¹] handelt es sich um difunktionelle Makromonomere, die zur Vernetzung der Copolymere geeignet sind.

Besonders bevorzugt als Makromonomere F) sind acrylisch- oder methacrylisch monofunktionalisierte Alkylethoxylate gemäß Formel (IV). 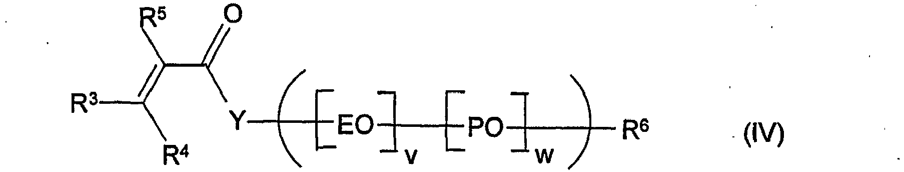 R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.

Bevorzugt sind R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest.

v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken.

Weiterhin insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ^{®}LA-030-methyacrylat | H | H | -CH₃ | -Lauryl | 3 | 0 |
| ^{®}LA-070-methacrylat | H | H | -CH₃ | -Lauryl | 7 | 0 |
| ^{®}LA-200-methacrylat | H | H | -CH₃ | -Lauryl | 20 | 0 |
| ^{®}LA-250-methacrylat | H | H | -CH₃ | -Lauryl | 25 | 0 |
| ^{®}T-080-methyacrylat | H | H | -CH₃ | -Talk | 8 | 0 |
| ^{®}T-080-acrylat | H | H | H | -Talk | 8 | 0 |
| ^{®}T-250-methyacrylat | H | H | -CH₃ | -Talk | 25 | 0 |
| ^{®}T-250-crotonat | -CH₃ | H | -CH₃ | -Talk | 25 | 0 |
| ^{®}OC-030-methacrylat | H | H | -CH₃ | -Octyl | 3 | 0 |
| ^{®}OC-105-methacrylat | H | H | -CH₃ | -Octyl | 10 | 5 |
| ^{®}Behenyl-010-methyaryl | H | H | H | -Behenyl | 10 | 0 |
| ^{®}Behenyl-020-methyaryl | H | H | H | -Behenyl | 20 | 0 |
| ^{®}Behenyl-010-senecionyl | -CH₃ | -CH₃ | H | -Behenyl | 10 | 0 |
| ^{®}PEG-440-diacrylat | H | H | H, | -Acryl | 10 | 0 |
| ^{®}B-11-50-methacrylat | H | H | -CH₃ | -Butyl | 17 | 13 |
| ^{®}MPEG-750-methacrylat | H | H | -CH₃ | -Methyl | 18 | 0 |
| ^{®}P-010-acrylat | H | H | H | -Phenyl | 10 | 0 |
| ^{®}O-050-acrylat | H | H | H | -Oleyl | 5 | 0 |

Weiterhin als Makromonomere F) insbesondere geeignet sind Ester der (Meth)acrylsäure mit
(C₁₀-C₁₈)-Fettalkoholpolyglykolethern mit 8 EO-Eiriheiten (Genapo^{®} C-080)
C₁₁-Oxoalkoholpolyglykolethern mit 8 EO-Einheiten (Genapol^{®} UD-080)
(C₁₂-C₁₄)-Fettalkoholpolyglykolethern mit 7 EO-Einheiten (Genapol^{®} LA-070)
(C₁₂-C₁₄)-Fettalkoholpolyglykolethern mit 11 EO-Einheiten (Genapol^{®} LA-110)
(C₁₆-C₁₈)-Fettalkoholpolyglykolethern mit 8 EO-Einheiten (Genapol^{®} T-080)
(C₁₆-C₁₈)-Fettalkoholpolyglykolethern mit 15 EO-Einheiten (Genapol^{®} T-150)
(C₁₆-C₁₈)-Fettalkoholpolyglykolethern mit 11 EO-Einheiten (Genapol^{®} T-110)
(C₁₆-C₁₈)-Fettalkoholpolyglykolethern mit 20 EO-Einheiten (Genapol^{®} T-200)
(C₁₆-C₁₈)-Fettalkoholpolyglykolethern mit 25 EO-Einheiten (Genapol^{®} T-250)
(C₁₈-C₂₂)-Fettalkoholpolyglykolethern mit 25 EO-Einheiten und/oder
iso-(C₁₆-C₁₈)-Fettalkoholpolyglykolethern mit 25 EO-Einheiten.

Bei den Genapol^{®}-Typen handelt es sich um Produkte der Firma Clariant GmbH.

Bevorzugt beträgt das Molekulargewicht der Makromonomeren F) 200 g/mol bis 10⁶ g/mol, besonders bevorzugt 150 bis 10⁴ g/mol und insbesondere bevorzugt 200 bis 5000 g/mol.

Bezogen auf die Gesamtmasse der Copolymere können geeignete Makromonomere bis zu 99,9 Gew.-% eingesetzt werden. Bevorzugt finden die Bereiche 0,5 bis 30 Gew.-% und 70 bis 99,5 Gew.-% Anwendung. Besonders bevorzugt sind Anteile von 1 bis 20 Gew.-% und 75 bis 95 Gew.-%.

Die Copolymerisation wird in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.

Die Additive G) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropfcopolymerisation statistisch von aktiven Radikalen angegriffen. Dies führt dazu, dass je nach Additiv G) mehr oder weniger große Anteile davon in die Copolymere eingebaut werden. Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.

Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen.

Besonders als Additive G) sind Homo- und Copolymere aus N-Vinylformamid, N-Vinylpyrrolidon, N-Vinylcaprolactam,

Insbesondere als Additive G) sind Pölyvinylpyrrolidone (z.B. Luviskol K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können. Das Molekulargewicht der Additive G) beträgt bevorzugt 10² bis 10⁷ g/mol, besonders bevorzugt 0,5*10⁴ bis 10⁶ g/mol.

Die Einsatzmenge des polymeren Additivs G) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.

Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.

Insbesondere bevorzugt als Vernetzer ist Trimethylolpropantriacrylat (TMPTA). Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.% und insbesondere bevorzugt 0,1 bis 7 Gew.%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodüsobutyronitril (AIBN), verwendet werden.

Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser/Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).

Bevorzugt eignet sich die Fällungspolymerisation, besonders bevorzugt die Fällungspolymerisation in tert.-Butanol.

Die nachfolgende Auflistung zeigt 67 Copolymere, die für die Formylierung der erfindungsgemäßen Mittel besonders geeignet sind. Die verschiedenen Copolymere Nr. 1 bis Nr. 67 sind gemäß den folgenden Herstellverfahren 1, 2, 3 und 4 erhältlich.

### Verfahren 1:

Diese Polymere sind nach dem Fällungsverfahren in tert. Butanol herstellbar.

Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere werden nach beendeter Reaktion (2 Stunden) durch Absaugen des Lösungsmittels und durch anschließende Vakuumtrocknung isoliert.

### Verfahren 2:

Diese Polymere sind nach dem Gelpolymerisationsverfahren in Wasser herstellbar.

Dabei werden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt Na₂S₂O₈) gestartet. Die Polymergele werden anschließend zerkleinert und nach Trocknung die Polymere isoliert.

### Verfahren 3:

Diese Polymere sind nach dem Emulsionsverfahren in Wasser herstellbar. Dabei werden die Monomere in einer Mischung aus Wasser/organ. Lösungsmittel (bevorzugt Cyclohexan) unter Verwendung eines Emulgators emulgiert, die Reaktionsmischung mittels N₂ inertisiert und anschließend die Reaktion nach Anheizen auf 80°C durch Zugabe von geeigneten Initiatoren- oder Initiatorsystemen (bevorzugt Na₂S₂O₈) gestartet. Die Polymeremulsionen werden anschließend eingedampft (Cyclohexan fungiert als Schlepper für Wasser) und dadurch die Polymere isoliert.

### Verfahren 4:

Diese Polymere sind nach dem Lösungsverfahren in organischen Lösungsmitteln (bevorzugt Toluol, z.B. auch tert. Alkohole) herstellbar. Dabei werden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere werden durch Abdampfen des Lösungsmittels und durch anschließende Vakuumtrocknung isoliert.

**Polymere mit hydrophoben Seitenketten, unvernetzt**

| Nr. | Zusammensetzung | Herstellverfahren |
|---|---|---|
| 1 | 95 g AMPS 5 g Genapol T-080 | 1 |
| 2 | 90 g AMPS 10 g Genapol T-080 | 1 |
| 3 | 85 g AMPS 15 g Genapol T-080 | 1 |
| 4 | 80 g AMPS 20 g Genapol T-080 | 1 |
| 5 | 70 g AMPS 30 g Genapol T-080 | 1 |
| 6 | 50 g AMPS 50 g Genapol T-080 | 3 |
| 7 | 40 g AMPS 60 g Genapol T-080 | 3 |
| 8 | 30 g AMPS 70 g Genapol T-080 | 3 |
| 9 | 20 g AMPS 80 g Genapol T-080 | 3 |
| 10 | 60 g AMPS 60 g BB10 | 4 |
| 11 | 80 g AMPS 20 g BB10 | 4 |
| 12 | 90 g AMPS 10 g BB10 | 3 |
| 13 | 80 g AMPS 20 g BB10 | 1 |
| 14 | 80 g AMPS 20 g Genapol LA040 | 1 |

**Polymere mit hydrophoben Seitenketten, vernetzt**

| Nr. | Zusammensetzung | Herstellverfahren |
|---|---|---|
| 15 | 80 g AMPS 20 g Genapol LA040 0.6g AMA | 1 |
| 16 | 80 g AMPS 20 g Genapol LA040 0,8 AMA | 1 |
| 17 | 80 g AMPS 20 g Genapol LA040 1,0 g AMA | 1 |
| 18 | 628,73 g AMPS 120,45 g Genapol T-250 6,5 g TMPTA | 2 |
| 19 | 60 g AMPS 40 g BB10 1,9 g TMPTA | 4 |
| 20 | 80 g AMPS 20 g BB10 1,4 g TMPTA | 4 |
| 21 | 90 g AMPS 10 g BB10 1,9 g TMPTA | 4 |
| 22 | 80 g AMPS 20 g BB10 1,9 g TMPTA | 4 |
| 23 | 60 g AMPS 40 g BB10 1,4 g TMPTA | 4 |

**Polymere mit hydrophoben Seitenketten, vernetzt, gepfropft**

| Nr. | Zusammensetzung | Hersteliverfahren |
|---|---|---|
| 24 | 95 g AMPS 5 g BB10, 1,9 g TMPTA, 1 g Poly-NVP | 1 |
| 25 | 90 g AMPS 10 g BB10, 1,9 g TMPTA, 1 g Poly-NVP | 1 |
| 26 | 85 g AMPS 15 g BB10, 1,9 g TMPTA, 1 g Poly-NVP | 1 |
| 27 | 90 g AMPS 10 g BB10, 1,9 g TMPTA, 1 g Poly-NVP | 1 |

**Polymere mit siliziumhaltigen Gruppen, unvernetzt**

| Nr. | Zusammensetzung | Herstellverfahren |
|---|---|---|
| 28 | 80 g AMPS, 20 g Silvet 867 | 1 |
| 29 | 80 g AMPS, 50 g Silvet 867 | 4 |

**Polymere mit siliziumhaltigen Gruppen, vernetzt**

| Nr. | Zusammensetzung | Herstellverfahren |
|---|---|---|
| 30 | 80 g AMPS, 20 g Silvet 867, 0,5 g MBA | 4 |
| 31 | 80 g AMPS, 20 g Silvet 867, 1,0 g MBA | 1 |
| 32 | 60 g AMPS, 40 g Y-12867, 0,95 g AMA | 1 |
| 33 | 80 g AMPS, 20 g Y-12867, 0,95 g AMA | 1 |
| 34 | 90 g AMPS, 10 g Y-12867, 0,95 g AMA | 1 |
| 35 | 60 g AMPS, 40 g Silvet 7280, 0,95 g AMA | 1 |
| 36 | 80 g AMPS, 20 g Silvet 7280, 0,95 g AMA | 1 |
| 37 | 90 g AMPS, 10 g Silvet 7280, 0,95 g AMA | 1 |
| 38 | 60 g AMPS, 40 g Silvet 7608, 0,95 g AMA | 1 |
| 39 | 80 g AMPS, 20 g Silvet 0,95 g AMA | 1 |
| 40 | 90 g AMPS, 10 g Silvet 7608, 0,95 g AMA | 1 |

**Polymere mit hydrophoben Seitenketten und kationischen Gruppen, unvernetzt**

| Nr. | Zusammensetzung | Herstellverfahren |
|---|---|---|
| 41 | 87,5 g AMPS, 7,5 g Genapol T-110, 5 g DADMAC | 2 |
| 42 | 40 g AMPS, 10 g Genapol T110, 45 g Methacrylamid | 2 |
| 43 | 55 g AMPS, 40 g Genapol LA040, 5 g Quat | 1 |
| 44 | 75 g AMPS, 10 g BB10, 6,7 g | 1 |

**Polymere mit hydrophoben Seitenketten und kationischen Gruppen, vernetzt**

| Nr. | Zusammensetzung | Herstellverfahren |
|---|---|---|
| 45 | 60 g AMPS, 20 g Genapol T-80, 10 g Quat, 10 g HEMA | 1 |
| 46 | 75 g AMPS, 20 g GenapolT-250, 5 g Quat, 1,4 g TMPTA | 1 |
| 47 | 75 g AMPS, 20 g GenapolT-250, 10 g Quat, 1,4 g TMPTA | 1 |
| 48 | 75 g AMPS, 20 g GenapolT-250, 20 g Quat, 1,4 g TMPTA | 1 |

**Polymere mit fluorhaltigen Gruppen**

| Nr. | Zusammensetzung | Herstellverfahren |
|---|---|---|
| 49 | 94 g AMPS, 2,02 g Fluowet AC 600 | 1 |
| 50 | 80 g AMPS, 20 g Perfluoroctylpolyethylenglykolmethacrylat, 1 g Span 80 | 3 |

**Polymere mit fluorhaltigen Gruppen, gepfropft**

| Nr. | Zusammensetzung | Herstellverfahren |
|---|---|---|
| 51 | 80 g AMPS, 10 g Fluowet AC 600, 5 g Poly-NVP | 1 |
| 52 | 70 g AMPS, 8 g Perfluoroctylethyloxyglycerinmethacrylat, 5 g Poly-NVP | 4 |

**Multifunktionelle Polymere**

| Nr. | Zusammensetzung | Herstellverfahren |
|---|---|---|
| 53 | 80 g AMPS, 10 g Genapol LA070, 10 g Silvet 7608, 1,8 g TMPTA | 1 |
| 54 | 70 g AMPS, 5 g N-Vinylpyrrolidon, 15 g Genapol T-250 Methacrylat, 10 g Quat, 10 g Poly-NVP | 4 |
| 55 | 80 g AMPS, 5 g N-Vinylformamid, 5 g Genapol O-150-methacrylat, 10 g DADMAC, 1,8 g TMPTA, 8 g Poly-N-Vinylformamid | 2 |
| 56 | 70 g AMPS, 5 g N-Vinylpyrrolidon, 15 g Genapol T-250-methacrylat, 10 g Quat, 10 g Poly-NVP | 1 |
| 57 | 60 g AMPS, 10 g Genapol-BE-020-methacrylat, 10 g Genapol T-250-acrylat, 20 g Quat, 1 g Span 80 | 1 |
| 58 | 60 g AMPS, 20 g MPEG-750-methacrylat, 10 g Methacryloxypyldimethicon, 10 g Perfluorooctylpolyethylenglycol-methacrylat, 10 g Poly[N-vinylcaprolacton-co-acrylsäure] (10/90) | 1 |
| 59 | 80 g AMPS, 5 g N-Vinylformamid, 5 g Genapol O-150-methacrylat, 10 g DADMAC, 1,8 g TMPTA | 1 |
| 60 | 70 g AMPS, 10 g Genapol T-250-acrylat, 5 g N-Methyl-4-vinylpyridiniumchlorid, 2,5 g Silvet Y-12867, 2,5 g Perfluorhexylpolyethylenglykolmethacrylat, 10 g Polyethylenglykoldimethacrylat, 4 g Poly[N-Vinylcaprolactam] | 1 |
| 61 | 10 g AMPS, 20 g Acrylamid, 30 g N-2-Vinylpyrrolidon, 20 g Silvet 7608, 10 g Methacryloxypyl dimethicon, 10 g Fluowet AC 812 | 3 |
| 62 | 60 g AMPS, 10 g DADMAC, 10 g Quat, 10 g Genapol-LA-250-crotonat, 10 g Methacryloxypyldimethicon, 7 g Poly[acrylsäure-co-N-vinylformamid] | 1 |
| 63 | 50 g AMPS, 45 g Silvet 7608, 1,8 g TMPTA, 8 g Poly[N-Vinylformamid] | 1 |
| 64 | 20 g AMPS, 10 g Genapol T 110, 35 g MAA, 30 g HEMA, 5 g DADMAC | 4 |
| 65 | 20 g AMPS, 80 g BB10, 1,4 g TMPTA | 1 |
| 66 | 75 g AMPS, 20 g BB10, 6,7 g Quat, 1,4 g TMPTA | 1 |
| 67 | 35 g AMPS, 60 g Acrylamid, 2 g VIFA, 2,5 g Vinylphosphonsäure, 2 Mol% Fluowet EA-600 | 4 |

**Chemische Bezeichnung der Reaktanden:**

| | |
|---|---|
| AMPS | Acryloyldimethyltaurat, wahlweise Na- oder NH4-Salz |
| Genapol^{®} T-080 | C₁₆-C₁₈-Fettalkoholpolyglykolether mit 8 EO-Einheiten |
| Genapol^{®} T-110 | C₁₂-C₁₄-Fettalkoholpolyglykolether mit 11 EO-Einheiten |
| Genapol^{®} T-250 | C₁₆-C₁₈-Fettalkoholpolyglycolether mit 25 EO-Einheiten |
| Genapol^{®} LA-040 | C₁₂-C₁₄-Fettalkoholpolyglykolether mit 4 EO-Einheiten |
| Genapol^{®} LA-070 | C₁₂-C₁₄-Fettalkoholpolyglykolether mit 7 EO-Einheiten |
| Genapol^{®} O-150 methacrylat | C₁₆-C₁₈-Fettalkoholpolyglykolether Methacrylat mit 15 EO-Einheiten, |
| Genapol^{®} LA-250 crotonat | C₁₂-C₁₄-Fettalkoholpolyglykolether crotonat mit 25 EO-Einheiten |
| Genapol^{®} T-250 methacrylat | C₁₆-C₁₈-Fettalkoholpolyglycolether methacrylat mit 25 EO-Einheiten |
| Genapol^{®} T-250 acrylat | C₁₆-C₁₈-Fettalkoholpolyglycolether methacrylat mit 25 EO-Einheiten |
| BB10^{®} | Polyoxyethylen(10)Behenylether |
| TMPTA | Trimethylolpropantriacrylat |
| Poly-NVP | Poly-N-Vinylpyrrolidon |
| Silvet^{®} 867 | Siloxan Polyalkylenoxid Copolymer |
| MBA | Methylen-bis-acrylamid |
| AMA | Allylmethacrylat |
| ^{®}Y-12867 | Siloxan Polyalkylenoxid Copolymer |
| Silvet^{®} 7608 | Polyalkylenoxid-modifiziertes Heptamethyltrisiloxan |
| Silvet^{®} 7280 | Polyalkylenoxid-modifiziertes Heptamethyltrisiloxan |
| DADMAC | Diallyldimethyl-ammoniumchlorid |
| HEMA | 2-Hydroxyethylmethacrylat |
| Quat | 2-(Methacryloyloxy)ethyltrimethylammoniumchlorid |
| Fluowet^{®} AC 600 | Perfluoralkylethylacrylat |
| Span^{®} 80 | Sorbitanester |

In einer bevorzugten Ausführungsform sind die Copolymere wasserlöslich oder wasserquellbar.

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbare Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden.

Die beschriebene, optional durchführbare Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften ist insbesondere bei der Verwendung als Leaveon Produkte, insbesondere im Falle von Emulsionen, gewünscht.

Vorteilhafte Eigenschaften zeigen die Copolymere sowohl in vernetzter als auch in unvernetzter Form. Während vernetzte Systeme z.B. hervorragende Eigenschaftsprofile im Hinblick auf Emulgier- und Dispergiervermögen und Emulsionsstabilisierung zeigten, konnten insbesondere mit Hilfe der quaternierten, Fluor- und siliziumhaltigen Varianten eine gute sensorische, glättende Wirkung der Mittel erzielt werden. Besonders vorteilhaft ist, dass die Copolymere überschüssiges Hautfett gut aufnehmen bei gleichzeitiger dauerhafter Haftwirkung der Mittel.

Die Copolymere können für dekorative kosmetische und dermatologische Mittel auf wässriger oder wässrig-alkoholischer Basis, in Öl-in-Wasser - und Wasser-in-Öl-Emulsionen und Suspensionen, Mikroemulsionen, sowie als Gleitmittel und Haftmittel in Lacken, Pudern und Pasten eingesetzt werden. Dabei können auch Mischungen der Copolymere verwendet werden.

Die erfindungsgemäßen Mittel enthalten bevorzugt 0,01 bis 10 Gew.-%, besonders bevorzugt 0,1 bis 5 Gew.-%, insbesondere bevorzugt 0,5 bis 3 Gew.-%, an Copolymeren.

Die erfindungsgemäßen Mittel können als weitere Hilfs- und Zusatzstoffe Pigmente und andere pulverförmige Stoffe, Ölkörper, Emulgatoren und Co-Emulgatoren, kationische Polymere, Filmbildner, Antioxidantien, Lichtschutzstoffe UV-Lichtschutzfilter, deodorierende Stoffe antimikrobiell wirkende Agentien Überfettungsmittel, feuchtigkeitsspendende Mittel, Stabilisatoren, biogene Wirkstoffe, Glycerin, Konservierungsmittel, Perlglanzmittel, Duftstoffe, Lösungsmittel, feuchtigkeitsspendende Mittel, Trübungsmittel, weitere Verdickungsmittel und Dispergiermittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, Erfrischungsmittel, beispielsweise Methylacetat, Stoffe mit keratolytischer und keratoplastischer Wirkung, Enzyme und Trägersubstanzen enthalten.

Als Pigmente eignen sich Metalloxide, beispielsweise Eisenoxide, Glimmer-Eisenoxid, Titanoxid, Glimmer-Titanoxid, Ultramarinblau, Chromoxide, sowie mit kationische Coatinghüllen modifizierte Pigmente, wie in WO 00/12053 und EP 504 066 beschrieben. Des weiteren können SiO₂, Silica, ZnO, Kaolin, mit SiO₂ modifiziertem Kaolin, Polytetrafluorethylen, Nylon, Talkum, Glimmer, Polymethylmethacrylat, Polyethylen, natürliche organische Verbindungen wie verkapselte oder unverkapselte Getreidestärke und Gemische davon eingesetzt werden.

Unter Ölkörper ist jegliche Fettsubstanz zu verstehen, die bei Raumtemperatur (25°C) flüssig ist.

Die Fett-Phase kann daher ein oder mehrere Öle umfassen, die vorzugsweise aus folgenden Ölen ausgewählt werden:
Silikonöle, flüchtig oder nicht flüchtig, linear, verzweigt oder ringförmig, eventuell organisch modifiziert; Phenylsilikone; Silikonharze und -gummis; Mineralöle wie Paraffin- oder Vaselinöl; Öle tierischen Ursprungs wie Perhydrosqualen, Lanolin; Öle pflanzlichen Ursprungs wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;

Synthetische Öle wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren; Ester wie Dioctyladipat, Diisopropyl dimer dilineloat; Propylenglykole/-dicaprilat oder Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle; fluorierte Silikonöle; Gemische der vorgenannten Verbindungen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate,

An kationische Polymere stehen die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Pölyquaternium-11, sowie Polyquaternium 37&mineral oil&PPG trideceth (Salcare SC95), PVP-dimethylaminoethylmethacrylat-Copolymer, Guarhydroxypropyltriammoniumchloride, sowie Calciumalginat und Ammoniumalginat zur Verfügung. Ebenso geeignet sind kationische Cellulosederivate; kationische Stärke; Copolymere von Diallylammoniumsalzen und Acrylamiden; quaternierte Vinylpyrrolidon/ Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5104 645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C₁₀-Polycarbamyl Polyglycerolester, Polyvinylalkohol, Polyvinylpyrrolidon, -copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Antioxidantien eignen sich beispielsweise Superoxid-Dismutase, Tocopherol (Vitamin E) und Ascorbinsäure (Vitamin C).

Als UV-Filter in Betracht kommen 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylenditmethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäure-isoamylester; 2,4,6-Tris-[p-(2-ethylhexyloxycarbonyl)anilino)-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; Octylmethoxycinnamat 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Als feuchtigkeitsspendende Substanz stehen beispielsweise Isopropylpalmitat, Glycerin und/ oder Sorbitol zu Verfügung.

Unter biogenen Wirkstoffen sind beispielsweise Pflanzenextrakte und Vitaminkomplexe zu verstehen.

Als Konservierungsmittel eignen sich beispielsweise Phenoxyethanol,

Diazolidinylharnstoff, Parabene, Pentandiol oder Sorbinsäure.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z.B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Als deodorierende Stoffe können z.B. Allantoin und Bisabolol in Gewichtsmengen von 0,0001 % bis 10 % eingesetzt werden.

Als antifungizide Wirkstoffe eignen sich bevorzugt Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Oczopyrox.

Als Verdickungs- und Dispergiermittel besonders geeignet sind Ethylenglykolester von Fettsäuren mit 14 bis 22, besonders bevorzugt 16 bis 22, Kohlenstoffatomen, insbesondere Mono- und Di-ethylenglykolstearat. Ebenfalls bevorzugt geeignet sind Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-amidobenzoesäure und deren lösliche Salze, N,N-Dihydrocarbyl-(C₁₆-C₁₈)-amidobenzoesäure und deren lösliche Salze und N,N-di(C₁₆-C₁₈)-amidobenzoesäure und deren Derivate. Weiterhin besonders geeignet sind Polyacrylate und Carbomere, insbesondere solche wasserlöslicher oder wasserquellbarer Copolymerisate auf Basis von Acrylamidoalkylsulfonsäuren und N-Vinylcarbonsäureamiden.

Als Solubilisatoren eignen sich prinzipiell alle ein- oder mehrwertigen Alkohole und ethoxylierten Alkohole. Bevorzugt werden Alkohole mit 1 bis 4 Kohlenstoffatomen, wie z.B. Ethanol, Propanol, Isopropanol, n-Butanol und Iso-Butanol, Glycerin und deren Mischungen eingesetzt. Weiterhin bevorzugt sind Polyethylenglykole mit einer relativen Molekülmasse unter 2000. Insbesondere bevorzugt sind Polyethylenglykole mit einer relativen Molekülmasse zwischen 200 und 600 in Mengen bis zu 45 Gew.-% und Polyethylenglykole mit einer relativen Molekülmasse zwischen 400 und 600 in Mengen von 0,5 bis 15 Gew.-%. Weitere geeignete Lösungsmittel sind beispielsweise Triacetin (Glycerintriacetat) und 1-Methoxy-2-propanol.

Als Stabilisatoren können Metallsalze von Fettsäuren, wie z.B. Magnesium-, Aluminium- und/oder Zinkstearat eingesetzt werden.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Die Mittel besitzen üblicherweise einen pH Wert im Bereich 2 bis 12, bevorzugt 3 bis 8.

Bei den erfindungsgemäßen Mitteln kann es sich um die unterschiedlichsten kosmetischen und dermatologischen Zubereitungen handeln. Insbesondere kann es sich um Make-ups, Grundierungen, Gesichtspuder, Rouge, Mascara, Lidschatten, Eye-liner, Lippenstifte, Cremes, Haarfärbemittel, Sonnenschutzmittel, Nagellacke und colorierte Gele handeln.

Die nachfolgenden Beispiele und Anwendungen sollen die Erfindung näher erläutern, ohne sie jedoch darauf zu beschränken (bei allen Prozentangaben handelt es sich um Gewichtsprozent). Bei den in den Beispielen verwendeten Copolymeren handelt es sich um Vertreter der in der Beschreibung bereits aufgeführten besonders bevorzugten Copolymere Nr. 1 bis Nr. 67. Die Herstellung erfolgte nach den dort angegebenen Verfahren 1, 2, 3 oder 4 unter Verwendung der jeweils bevorzugten Initiatoren und Lösemittel.

### Beispiel 1: Kosmetische Grundlage

| | |
|---|---|
| Kaolin modifiziert mit SiO₂ | 2,5 % |
| Copolymer Nr. 66 | 0,4 % |
| Glycereth-26 | 4,0 % |
| Silica | 1,0 % |
| Di-C₁₂-C₁₃-alkylmalate | 11,0 % |
| Eisenoxide | 1,25 % |
| Titaniumoxid | 5,0 % |
| Neopentyl glycol diheptanoate | 3,0 % |
| Diethylen glycol dioctanoate/diisononanoat | 3,5 % |
| Tridecyl neopentanoate | 2,0 % |
| Tocopherol acetat | 0,2 % |
| Myristyl lactate | 2,0 % |
| Cyclomethicone & Dimethyconol | 1,0 % |
| Porphyridium Cruentum Extrakt | 5,0 % |
| Parfümöl | 0,4 % |
| Konservierungsmittel | 0,5 % |
| Deionisiertes Wasser | ad 100 |

### Herstellung:

Die Ölphase wird auf 80°C erwärmt, die Pigmente in Glycereth-25 oder PEG 8 zugegeben. Ebenso wird das Wasser auf 80°C erwärmt, AMPS-Copolymer zugegeben und die beiden Phasen bei 8000 U/Min emulgiert. Danach wurde das Produkt unter langsamem Rühren (etwa 200 U/Min) entlüftet und abgekühlt.

### Beispiel 2: Kosmetische Grundierung mit Sonnenschutz

| | |
|---|---|
| PEG-8 oder Glycereth-25 | 4,0 |
| Copolymer Nr. 41 | 0,4 |
| Eisenoxide | 1,25 |
| Titaniumoxid | 5,0 |
| Tocopherol acetat | 0,2 |
| C12-C13-Alkyloctanoat | 18,0 |
| Octylmethoxycinnamat | 7,0 |
| Konservierungsmittel | 0,5 |
| Deionisiertes Wasser | ad 100 |

### Herstellung:

Die Ölphase wird auf 80°C erwärmt, die Pigmente in Glycereth-25 oder PEG 8 zugegeben. Ebenso wird das Wasser auf 80°C erwärmt, Polyquaternium zugegeben und die beiden Phasen bei 8000 U/Min emulgiert. Danach wurde das Produkt unter langsamem Rühren (etwa 200 U/Min) entlüftet, bei ca. 60°C Octylmethoxycinnamat zugegeben und weiter langsam abgekühlt.

### Beispiel 3: Wimperntusche

| | |
|---|---|
| Polyvinylpyrrolidon | 4,0 |
| Copolymer Nr. 67 | 0,2 |
| Glycereth-26 | 2,0 |
| Triethanolamin 99% | 2,4 |
| Magnesium-Aluminium-Silikat | 1,0 |
| Talkum | 1,0 |
| Weizenkeimöl | 1,0 |
| PVP/Eicosene Copolymer | 2,0 |
| Eisenoxide | 12,0 |
| Hydrogeniertes Polyisobuten | 0,2 |
| Cetearylalkohol | 0,1 |
| Stearinsäure | 4,0 |
| Carnauba | 4,0 |
| Sorbitansesquioleat | 1,3 |
| Bienenwachs | 4,0 |
| C₁₈-C₃₆-Triglycerid | 8,5 |
| Lecithin | 1,0 |
| Tocopherolacetat | 0,2 |
| Konservierungsmittel | 0,7 |
| Deionisiertes Wasser | ad 100 |

Die Gele und Polymere wurden in der wässrigen Phase dispergiert. Anschließend wurden die Pigmente dispergiert. Die Fertigstellung der Emulsion erfolgte bei 85°C.

### Beispiel 4: Make-up

| | | |
|---|---|---|
| A | Cyclomethicon | 20,00 |
| | Copolymer Nr. 32 | |
| | with Cyclomethicon | 9,00 |
| B | Titandioxid | 8,33 |
| | Eisenoxid, gelb | 1,35 |
| | Eisenoxid, rot | 0,26 |
| | Eisenoxid, schwarz | 0,06 |
| | Zinkoxid | 5,00 |
| C | Phenylbenzimidazolsulfonsäure | 5,20 |
| | Deionisiertes Wasser | 1,80 |
| | Triethanolamin | 3,00 |
| | Polysorbate-20 | 0,05 |
| | Deionisiertes Wasser | ad 100 |
| D | Methylparaben | 0,50 |
| E | Silica | 1,00 |

### Herstellung:

B wurde unter Rühren bei Raumtemperatur zu A gegeben, dann C, anschließend D und E zu den homogenen Gemischen hinzugefügt.

## Patentansprüche

1. Verwendung von kosmetischen und dermatologischen Mitteln, **dadurch gekennzeichnet, dass** sie mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) einer oder mehreren mindestens monofunktionellen, zur radikalischen Polymerisation befähigten, siliziumhaltigen Komponente(n), wobei mindestens eine siliziumhaltige Komponente eine Verbindung ausgewählt aus den Formeln 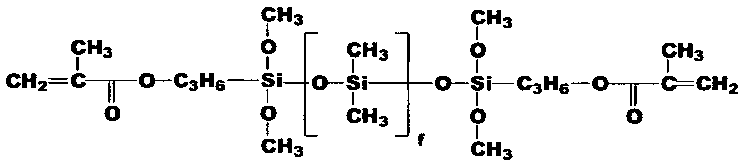 worin f = 2 bis 500 bedeutet, 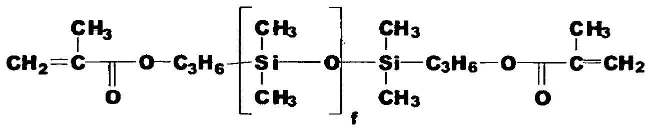 worin f = 2 bis 500 bedeutet, und 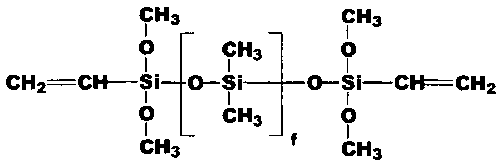 worin f = 2-500 bedeutet, ist,
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation in Gegenwart mindestens eines polymeren Additivs mit zahlenmittleren Molekulargewichten von 200 g/mol bis 10⁹ g/mol ausgewählt aus Polyvinylpyrrolidonen, Poly(N-Vinylform-amiden), Poly(N-Vinylcaprolactemen) und Copolymeren aus N-Vinylpyrrolidon, N-Vinylformamid und/oder Acrylsäure, erfolgt,
enthalten, als dekorative Mittel.

2. Verwendung nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Verwendung nach Anspruch 1 und/oder 2, **dadurch gekennzeichnet, dass** es sich beiden Comonomeren C) um Diallyldimethylammoniumchlorid (DADMAC),
[2-((Methacryloyloxy)ethyl]trimethylammoniumchlorid (MAPTAC),
[2-((Acryloyloxy)ethyl]trimethylammoniumchlorid,
[2-Methacrylamidoethyl]trimethylammoniumchlorid,
[2-((Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain handelt.

4. Verwendung nach mindestens einem der Ansprüche 1 bis 3, **dadurch gekennzeichnet, dass** es sich bei den siliziumhaltigen Komponenten D) um weitere Verbindungen der Formel (I)
R¹ - Z- [(Si(R³R⁴)-O-)_{w}-(Si(R⁵R⁶)-O)ₓ-]-R² (I)
handelt, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁-C₅₀) Alkylen)-, -((C₆-C₃₀) Arylen)-, -((C₅-C₈) Cycloalkylen)-, -((C₁-C₅₀) Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, -((C₁-C₁₀) Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe -Z-R¹ bedeutet, wobei Z und R¹ die oben genannten Bedeutungen haben und R⁷ eine Gruppe der Formel -O-Si(CH₃)₃, -O-Si(Phenyl)₃, -O-Si(O-Si(CH₃)₃)₂CH₃) und -O-Si(O-Si(Ph)₃)₂Ph) bedeutet.

5. Verwendung nach mindestens einem der Ansprüche 1 bis 4, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt -O-, -C(O)-, -C(O)-O-, -S-, -O-CH₂-CH(O-)-CH₂OH, -O-CH₂-CH(OH)-CH₂-O-, -O-SO₂-O- , -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten, darstellt und
r, s stöchiometrische Koeffizienten darstellen, die unabhängig voneinander Zahlen zwischen 0 und 200 sein können.

6. Verwendung nach mindestens einem der Ansprüche 1 bis 5, **dadurch gekennzeichnet, dass** es sich bei den Makromonomeren F) um Verbindungen der Formel (III) handelt,
R¹-Y-[(A)v-(B)_{w}-(C)ₓ-(D)_{z}]-R² (III)
wobei R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine verbrückende Gruppe, bevorzugt -O-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)- darstellt;
A, B, C und D unabhängig voneinander diskrete chemische Wiederholungseinheiten, bevorzugt hervorgegangen aus Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid, insbesondere bevorzugt Ethylenoxid, Propylenoxid darstellen;
v, w, x und z unabhängig voneinander 0 bis 500, bevorzugt 1 bis 30, betragen, wobei die Summe aus v, w, x und z im Mittel ≥1 ist; und
R² einen linearen oder verzweigten aliphatischen, olefinischen, cycloaliphatischen, arylaliphatischen oder aromatischen (C₁-C₅₀)-Kohlenwasserstoffrest, OH, -NH₂ oder -N(CH₃)₂ darstellt oder gleich [-Y-R¹] ist.

7. Verwendung nach mindestens einem der Ansprüche 1 bis 6, **dadurch gekennzeichnet, dass** die Copolymerisation in Gegenwart mindestens eines polymeren Additivs G) erfolgt.

8. Verwendung nach mindestens einem der Ansprüche 1 bis 7, **dadurch gekennzeichnet, dass** die Copolymere vernetzt sind.

9. Verwendung nach mindestens einem der Ansprüche 1 bis 8, **dadurch gekennzeichnet, dass** die Copolymere durch Fällungspolymerisation in tert.-Butanol hergestellt werden.

10. Verwendung nach mindestens einem der Ansprüche 1 bis 9, **dadurch gekennzeichnet, dass** die Copolymere wasserlöslich oder wasserquellbar sind.

11. Verwendung nach mindestens einem der Ansprüche 1 bis 10, **dadurch gekennzeichnet, dass** die Mittel, bezogen auf die fertigen Mittel, 0,01 bis 10 Gew.-% der Copolymere enthalten.

12. Verwendung nach mindestens einem der Ansprüche 1 bis 11 **dadurch gekennzeichnet, dass** die Mittel Pigmente in Form von Metalloxiden, bevorzugt in Form von Eisenoxid, Glimmer-Eisenoxid, Titanoxid, Glimmer-Titanoxid, Ultramarinblau, Chromoxid, in Form von mit kationischen Coatinghüllen modifizierten Pigmenten, in Form von SiO₂, Silica, ZnO, Kaolin, mit SiO₂ modifiziertem Kaolin, Polytetrafluorethylen, Nylon, Talkum, Glimmer, Polymethylmethacrylat, Polyethylen enthalten.

13. Verwendung nach mindestens einem der Ansprüche 1 bis 12, **dadurch gekennzeichnet, dass** es sich bei den Mitteln um Make-ups, Grundierungen, Gesichtspuder, Rouge, Mascara, Lidschatten, Eye-liner, Lippenstifte, Cremes, Haarfärbemittel, Sonnenschutzmittel, Nagellacke oder colorierte Gele handelt.

## Claims

1. The use of cosmetic and dermatological compositions which comprise at least one copolymer obtainable by free-radical copolymerization of
A) acryloyldimethyltaurine and/or acryloyldimethyltaurates,
B) if desired, one or more further olefinically unsaturated, noncationic, optionally crosslinking, comonomers which have at least one oxygen, nitrogen, sulfur or phosphorus atom and possess a molecular weight of less than 500 g/mol,
C) if desired, one or more olefinically unsaturated, cationic comonomers which have at least one oxygen, nitrogen, sulfur or phosphorus atom and possess a molecular weight of less than 500 g/mol,
D) one or more at least monofunctional silicon-containing components capable of free-radical polymerization, at least one silicon-containing component being a compound selected from the formulae 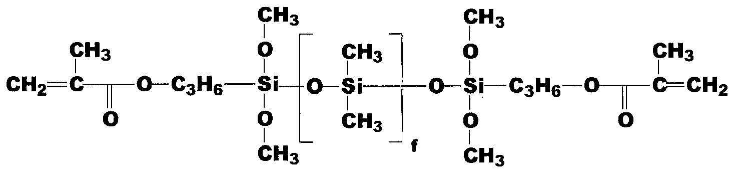 in which f = 2 to 500, 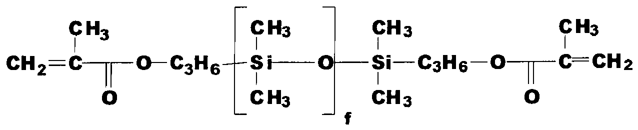 in which f = 2 to 500, and 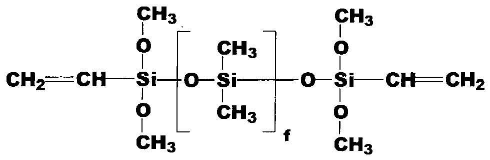 in which f = 2-500,
E) if desired, one or more fluorine-containing components capable of free-radical polymerization and having a functionality of at least one,
F) if desired, one or more olefinically mono- or polyunsaturated, optionally crosslinking macromonomers each possessing at least one oxygen, nitrogen, sulfur or phosphorus atom and having a number-average molecular weight of greater than or equal to 200 g/mol, the macromonomers not being a silicon-containing component D) or fluorine-containing component E),
G) the copolymerization taking place in the presence of at least one polymeric additive having number-average molecular weights of from 200 g/mol to 10⁹ g/mol selected from polyvinylpyrrolidones, poly(N-vinylformamides), poly(N-vinylcaprolactams), and copolymers of N-vinylpyrrolidone, N-vinylformamide and/or acrylic acid,
as decorative compositions.

2. The use as claimed in claim 1, wherein the comonomers B) are unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

3. The use as claimed in claim 1 and/or 2, wherein the comonomers C) are
diallyldimethylammonium chloride (DADMAC),
[2-((methacryloyloxy)ethyl]trimethylammonium chloride (MAPTAC),
[2-((acryloyloxy)ethyl]trimethylammonium chloride,
[2-methacrylamidoethyl]trimethylammonium chloride,
[2-((acrylamido)ethyl]trimethylammonium chloride,
N-methyl-2-vinylpyridinium chloride,
N-methyl-4-vinylpyridinium chloride,
dimethylaminoethyl methacrylate,
dimethylaminopropylmethacrylamide,
methacryloylethyl N-oxide and/or
methacryloylethylbetaine.

4. The use as claimed in at least one of claims 1 to 3, wherein the silicon-containing components D) are further compounds of the formula (I)
R¹ -Z- [(Si(R³R⁴)-O-)_{w}-(Si(R⁵R⁶)-O)x-]- R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably selected from -O-, -((C₁-C₅₀)alkylene)-, -((C₆-C₃₀)arylene)-, -((C₅-C₈) cycloalkylene) -, -((C₁-C₅₀) alkenylene) -, -(polypropylene oxide)ₙ-, -(polyethylene oxide)ₒ-, -(polypropylene oxide)ₙ(polyethylene oxide)ₒ-, where n and o independently of one another denote numbers from 0 to 200 and the distribution of the EO/PO units can be random or in the form of blocks,
-((C₁-C₁₀)alkyl)-(Si(OCH₃)₂)- and - (Si(OCH₃)₂) -;
R³, R⁴, R⁵, and R⁶ denote independently of one another -CH₃, -O-CH₃, -C₆H₅ or
-O-C₆H₅;
w and x denote numbers from 0 to 500, either w or x necessarily being greater than zero, and
R² is a saturated or unsaturated, aliphatic, cycloaliphatic, arylaliphatic or aromatic radical having in each case 1 to 50 carbon atoms or a group of the formulae -OH, -NH₂, -N(CH₃)₂ , -R⁷ or a group -Z-R¹, where Z and R¹ have the abovementioned definitions, and R⁷ is a group of the formula -O-Si(CH₃)₃, -O-Si (phenyl)₃,
-O-Si (O-Si(CH₃)₃)₂CH₃), and -O-Si(O-Si(Ph)₃)₂Ph).

5. The use as claimed in at least one of claims 1 to 4, wherein the fluorine-containing components E) are compounds of the formula (II)
R¹-Y-CᵣH₂ᵣCₛF₂ₛCF₃ (II)
where
R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a chemical bridge, preferably -O-, -C(O)-, - C(O)-O-, -S-,
-O-CH₂-CH(O-)-CH₂OH, -O-CH₂-CH(OH)-CH₂-O-, -O-SO₂-O-,
-O-S(O)-O-, -PH-, -P(CH₃)-, -PO₃-, -NH-, -N(CH₃)-, -O- (C₁-C₅₀) alkyl-O-, -N (CH₃)
-O-phenyl-O-, -O-benzyl-O-, -O-(C₅-C₈)cycloalkyl-O-,
-O-(C₁-C₅₀)alkenyl-O-, -O-(CH(CH₃) -CH₂-O)ₙ-, -O-(CH₂-CH₂-O)ₙ-, and
-O-([CH-CH₂-O]ₙ-[CH₂-CH₂-O]ₘ)ₒ-, where n, m, and o independently of one another denote numbers from 0 to 200, and
r, s are stoichiometric coefficients which independently of one another can be numbers between 0 and 200.

6. The use as claimed in at least one of claims 1 to 5, wherein the macromonomers F) are compounds of the formula (III)
R¹-Y- [(A)ᵥ-(B)_{w}-(C)ₓ-(D)_{z}]-R² (III)
where R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a bridging group, preferably -O-, -S-, -C(O)-, - C(O)-O-,
-O-CH₂-CH(O-)-CH₂OH, -O-CH₂-CH(OH)-CH₂O-, -O-SO₂-O-, -0-SO₂-O-,
-O-SO-O-, -PH-, -P(CH₃)-, -PO₃-, -NH-, and -N(CH₃) -;
A, B, C, and D independently of one another are discrete chemical repeating units, preferably originating from acrylamide, methacrylamide, ethylene oxide, propylene oxide, AMPS, acrylic acid, methacrylic acid, methyl methacrylate, acrylonitrile, maleic acid, vinyl acetate, styrene, 1,3-butadiene, isoprene, isobutene, diethylacrylamide, and diisopropylacrylamide, more preferably ethylene oxide or propylene oxide;
v, w, x, and z independently of one another amount to from 0 to 500, preferably from 1 to 30, the sum of v, w, x, and z being on average ≥ 1; and
R² is a linear or branched aliphatic, olefinic, cycloaliphatic, arylaliphatic or aromatic (C₁-C₅₀) hydrocarbon radical, OH, -NH₂ or -N(CH₃)₂ or is [-Y-R¹].

7. The use as claimed in at least one of claims 1 to 6, wherein the copolymerization takes place in the presence of at least one polymeric additive G).

8. The use as claimed in at least one of claims 1 to 7, wherein the copolymers are crosslinked.

9. The use as claimed in at least one of claims 1 to 8, wherein the copolymers are prepared by precipitation polymerization in tert-butanol.

10. The use as claimed in at least one of claims 1 to 9, wherein the copolymers are water-soluble or water-swellable.

11. The use as claimed in at least one of claims 1 to 10, wherein the compositions comprise, based on the finished compositions, from 0.01 to 10% by weight of the copolymers.

12. The use as claimed in at least one of claims 1 to 11, wherein the compositions comprise pigments in the form of metal oxides, preferably in the form of iron oxide, mica-iron oxide, titanium oxide, mica-titanium oxide, ultramarine blue, chromium oxide, in the form of pigments modified with cationic coating shells, in the form of SiO₂, silica, ZnO, kaolin, SiO₂-modified kaolin, polytetrafluoroethylene, nylon, talc, mica, polymethyl methacrylate, polyethylene.

13. The use as claimed in at least one of claims 1 to 12, wherein the compositions are makeups, foundations, face powders, rouge, mascara, eyeshadows, eyeliners, lipsticks, creams, hair colorants, sunscreens, nail lacquers or colored gels.

## Revendications

1. Utilisation d'agents cosmétiques et dermatologiques, **caractérisée en ce qu'**ils contiennent au moins un copolymère pouvant être obtenu par copolymérisation radicalaire de
A) de l'acide acryloyldiméthyltaurique et/ou des taurates d'acryloyldiméthyle,
B) éventuellement un ou plusieurs comonomères supplémentaires oléfiniquement insaturés, non cationiques, éventuellement réticulants, qui comprennent au moins un atome d'oxygène, d'azote, de soufre ou de phosphore et qui présentent un poids moléculaire inférieur à 500 g/mol,
C) éventuellement un ou plusieurs comonomères cationiques oléfiniquement insaturés, qui comprennent au moins un atome d'oxygène, d'azote, de soufre ou de phosphore et qui présentent un poids moléculaire inférieur à 500 g/mol,
D) un ou plusieurs composants contenant du silicium, au moins monofonctionnels, aptes à la polymérisation radicalaire, au moins un composant contenant du silicium étant un composé choisi parmi les formules 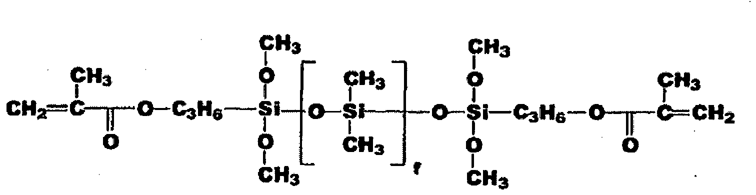 dans laquelle f = 2 à 500, 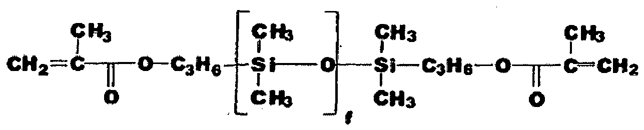 dans laquelle f = 2 à 500, et 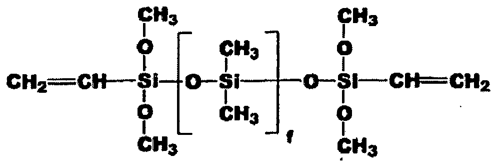 dans laquelle f = 2 à 500,
E) éventuellement un ou plusieurs composants contenant du fluor, au moins monofonctionnels, aptes à la polymérisation radicalaire,
F) éventuellement un ou plusieurs macromonomères mono- ou polyoléfiniquement insaturés, éventuellement réticulants, qui comprennent à chaque fois au moins un atome d'oxygène, d'azote, de soufre ou de phosphore et qui présentent un poids moléculaire moyen en nombre supérieur ou égal à 200 g/mol, les macromonomères n'étant pas un composant contenant du silicium D) ou un composant contenant du fluor E),
G) la copolymérisation ayant lieu en présence d'au moins un additif polymère de poids moléculaire moyen en nombre de 200 g/mol à 10⁹ g/mol choisi parmi les polyvinylpyrrolidones, les poly(N-vinylformamides), les poly(N-vinylcaprolactames) et les copolymères de N-vinylpyrrolidone, de N-vinylformamide et/ou d'acide acrylique,
en tant qu'agents décoratifs.

2. Utilisation selon la revendication 1, **caractérisée en ce que** les comonomères B) sont des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques de 1 à 22 atomes C, des N-vinylamides à chaîne ouverte, des N-vinylamides cycliques d'une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle ; le styrène, l'acrylonitrile, le chlorure de vinyle, le chlorure de vinylidène, le tétrafluoroéthylène, l'acide vinylphosphonique ou ses esters ou sels, l'acide vinylsulfonique ou ses esters ou sels, l'acide allylphosphonique ou ses esters ou sels et/ou l'acide méthallylsulfonique ou ses esters ou sels.

3. Utilisation selon la revendication 1 et/ou 2, **caractérisée en ce que** les comonomères C) sont le chlorure de diallyldiméthylammonium (DADMAC), le chlorure de [2-(méthacryloyloxy)éthyl]triméthylammonium (MAPTAC), le chlorure de [2-(acryloyloxy)éthyl]triméthylammonium, le chlorure de [2-méthacrylamidoéthyl]triméthylammonium, le chlorure de [2-(acrylamido)éthyl]triméthylammonium, le chlorure de N-méthyl-2-vinylpyridinium, le chlorure de N-méthyl-4-vinylpyridinium, le méthacrylate de diméthylaminoéthyle, le diméthylaminopropylméthacrylamide, le N-oxyde de méthacryloyléthyle et/ou la méthacryloyléthyl-bétaïne.

4. Utilisation selon au moins l'une quelconque des revendications 1 à 3, **caractérisée en ce que** les composants contenant du silicium D) sont des composés de formule (I) supplémentaires
R¹-Z-[(Si(R³R⁴)-O-)_{w}-(Si(R⁵R⁶)-O)ₓ-]-R² (I)
dans laquelle
R¹ représente un radical vinyle, allyle, méthallyle, méthylvinyle, acryle, méthacryle, crotonyle, sénécionyle, itaconyle, maléinyle, fumaryle ou styryle ;
Z représente un pont chimique, de préférence choisi parmi -O-, -(alkylène en (C₁-C₅₀))-, -(arylène en (C₆-C₃₀))-, -(cycloalkylène en (C₅-C₈))-, -(alcénylène en (C₁-C₅₀))-, -(oxyde de polypropylène)ₙ-, -(oxyde de polyéthylène)ₒ-, -(oxyde de polypropylène)ₙ(oxyde de polyéthylène)ₒ-, n et o signifiant indépendamment l'un de l'autre des nombres de 0 à 200 et la distribution des unités EO/PO pouvant être statistique ou séquencée, -(alkyle en (C₁-C₁₀))-(Si(OCH₃)₂)- et -(Si(OCH₃)₂)- ;
R³, R⁴, R⁵ et R⁶ signifient indépendamment les uns des autres -CH₃, -O-CH₃, -C₆H₅ ou -O-C₆H₅ ;
w, x signifient des nombres de 0 à 500, w ou x devant être supérieur à zéro, et
R² signifie un radical saturé ou insaturé, aliphatique, cycloaliphatique, arylaliphatique ou aromatique, contenant à chaque fois 1 à 50 atomes C, ou un groupe de formule -OH, -NH₂, -N(CH₃)₂, -R⁷ ou un groupe -Z-R¹, Z et R¹ ayant les significations indiquées précédemment, et R⁷ signifiant un groupe de formule -O-Si(CH₃)₃, -O-Si(phényle)₃, -O-Si(O-Si(CH₃)₃)₂CH₃) et -O-Si (O-Si(Ph)3)₂Ph).

5. Utilisation selon au moins l'une quelconque des revendications 1 à 4, **caractérisée en ce que** les composants contenant du fluor E) sont des composés de formule (II)
R¹-Y-CᵣH₂ᵣCₛF₂ₛCF₃ (II)
dans laquelle
R¹ représente une fonction polymérisable du groupe des composés vinyliquement insaturés, de préférence un radical vinyle, allyle, méthallyle, méthylvinyle, acryle, méthacryle, crotonyle, sénécionyle, itaconyle, maléinyle, fumaryle ou styryle ;
Y représente un pont chimique, de préférence -0-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-alkyle en (C₁-C₅₀)-O-, -O-phényl-O-, -O-benzyl-O-, -O-cycloalkyle en (C₅-C₈)-O-, -O-alcényle en (C₁-C₅₀)-O-, -O-(CH(CH₃)-CH₂-O)ₙ-, -O-(CH₂-CH₂-O)ₙ- et -O-([CH-CH₂-O]ₙ-[CH₂-CH₂-O]ₘ)ₒ-, n, m et o signifiant indépendamment les uns des autres des nombres de 0 à 200, et
r, s représentant des coefficients stoechiométriques qui peuvent être indépendamment l'un de l'autre des nombres compris entre 0 et 200.

6. Utilisation selon au moins l'une quelconque des revendications 1 à 5, **caractérisée en ce que** les macromonomères F) sont des composés de formule (III)
R¹-Y- [(A)ᵥ-(B)_{w}-(C)ₓ-(D)_{z}]-R² (III)
dans laquelle R¹ représente une fonction polymérisable du groupe des composés vinyliquement insaturés, de préférence un radical vinyle, allyle, méthallyle, méthylvinyle, acryle, méthacryle, crotonyle, sénécionyle, itaconyle, maléinyle, fumaryle ou styryle ;
Y représente un groupe pontant, de préférence -0-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- et -N(CH₃)- ;
A, B, C et D représentent indépendamment les uns des autres des unités de répétition chimiques discrètes, de préférence provenant d'acrylamide, de méthacrylamide, d'oxyde d'éthylène, d'oxyde de propylène, d'AMPS, d'acide acrylique, d'acide méthacrylique, de méthacrylate de méthyle, d'acrylonitrile, d'acide maléique, d'acétate de vinyle, de styrène, de 1,3-butadiène, d'isoprène, d'isobutène, de diéthylacrylamide et de diisopropylacrylamide, de manière particulièrement préférée d'oxyde d'éthylène, d'oxyde de propylène ;
v, w, x et z représentent indépendamment les uns des autres 0 à 500, de préférence 1 à 30, la somme de v, w, x et z étant en moyenne ≥ 1 ; et
R² représente un radical hydrocarboné en (C₁-C₅₀) linéaire ou ramifié, aliphatique, oléfinique, cycloaliphatique, arylaliphatique ou aromatique, OH, -NH₂ ou -N(CH₃)₂ ou [-Y-R¹].

7. Utilisation selon au moins l'une quelconque des revendications 1 à 6, **caractérisée en ce que** la copolymérisation a lieu en présence d'au moins un additif polymère G).

8. Utilisation selon au moins l'une quelconque des revendications 1 à 7, **caractérisée en ce que** les copolymères sont réticulés.

9. Utilisation selon au moins l'une quelconque des revendications 1 à 8, **caractérisée en ce que** les copolymères sont fabriqués par polymérisation par précipitation dans du tert.-butanol.

10. Utilisation selon au moins l'une quelconque des revendications 1 à 9, **caractérisée en ce que** les copolymères sont solubles dans l'eau ou gonflants dans l'eau.

11. Utilisation selon au moins l'une quelconque des revendications 1 à 10, **caractérisée en ce que** les agents contiennent, par rapport aux agents finis, 0,01 à 10 % en poids des copolymères.

12. Utilisation selon au moins l'une quelconque des revendications 1 à 11, **caractérisée en ce que** les agents contiennent des pigments sous la forme d'oxydes de métaux, de préférence sous la forme d'oxyde de fer, de mica-oxyde de fer, d'oxyde de titane, de mica-oxyde de titane, de bleu d'outremer, d'oxyde de chrome, sous la forme de pigments modifiés avec des enveloppes de revêtement cationiques, sous la forme de SiO₂, de silice, de ZnO, de kaolin, de kaolin modifié avec SiO₂, de polytétrafluoroéthylène, de nylon, de talc, de mica, de polyméthacrylate de méthyle, de polyéthylène.

13. Utilisation selon au moins l'une quelconque des revendications 1 à 12, **caractérisée en ce que** les agents sont des produits de maquillages, des fonds de teint, des poudres pour le visage, des blushs, des mascaras, des fards à paupières, des eye-liners, des rouges à lèvres, des crèmes, des produits colorants capillaires, des produits de protection solaire, des vernis à ongles ou des gels colorés.
